# EUROPEAN PATENT APPLICATION

(11) **EP 4 382 163 A1**
(43) Date of publication of application: **12.06.2024**
(21) Application number: 23215047.4
(22) Date of filing: 07.12.2023
(51) Int. Cl.: A61N 5/06

(54) **LIGHT TREATMENT DEVICES AND METHODS OF USE**

(30) Priority: 09.12.2022 US 202263431356 P; 27.04.2023 US 202318140149
(71) Applicant: Sedic, Filip, 114 31 Stockholm (SE)
(72) Inventor: Sedic, Filip, 114 31 Stockholm (SE)
(74) Representative: Marks & Clerk LLP

(57) **Abstract**

A treatment device (2) configured to be worn by a user, comprising:
a main body (10) having a first end (20), a second end (30) substantially opposite the first end (20), an intermediate portion (40) extending from the first end (20) to the second end (30), a frame (60), and a first portion (120) secured to the frame (60), the first portion (120) defining a set of passageways (110) extending from a first side (11) of the first portion (120) to a second side (12) of the first portion; and
a light source (100) housed within the main body (10) comprising a first set of lights and a second set of lights, wherein each of the first and second set of lights are operatively connected to the frame (60) and configured to emit light into the first portion (120) and through the set of passageways (110) defined by the first portion (120).

## Description

### FIELD

The disclosure relates generally to the field of light treatment devices and methods of use. More particularly, the disclosure relates to light treatment devices suitable for use on the human face, neck, and body and various methods of using the same.

### BACKGROUND

Light has been used to treat various mental and physical conditions for centuries. Skin health and appearance is an important aspect of many beauty regimens. As a person ages, his or her skin tends to develop wrinkles, sag, become rough, and/or develop various spots or marks. It is known that stimulating skin cells through various means such as through light therapy, can produce positive effects on the skin. Such positive effects, for example, may include one or more of: increased collagen production, reduction of skin inflammation, reduction of acne, improvement of blood flow, tightening of the skin, and/or the destruction of bacteria.

More specifically, various colors and/or wavelengths of light emitting diode ("LED") light are known to have particular benefits when used by a human. Red LED light us known to diminish signs of aging and make skin younger looking. Blue light is said to kill bacteria and/or reduce the appearance of blemishes. Green LED light (known to bright complexion and even skin tone), Orange LED light (helps skin glow), Purple LED light (reduction of age spots and hyperpigmentation), and Cyan LED light (calms skin breakouts, and Yellow LED light (reduces appearance of redness and soothes skin) all impact the skin differently. Moreover, near infrared light ("NIR light") improves skin that has been damaged by the sun and the appearance of fine lines, wrinkles, and sagging skin.

Several devices exist which may provide light therapy to a user. These devices are often located in medical or cosmetic offices and use of such devices requires the assistance of another person to administer the desired treatment. In many instances, an administrator of the therapy must be licensed by a state or federal board (such as a medical or cosmetology board) or receive a certification to use such devices. Alternatively, at-home devices, which typically comprise masks, do not efficiently and effectively distribute light to a user's skin.

A need exists, therefore, for improved skincare devices and methods of use that provide light treatment to a user.

### BRIEF SUMMARY OF SELECTED EXAMPLES

Various example treatment light treatment devices and methods of use are described.

An example treatment device configured to be worn by a user comprises a main body having a first end, a second end substantially opposite the first end, an intermediate portion extending from the first end to the second end, a frame, and a first portion disposed secured to the frame, the first portion defining a set of passageways extending from a first side of the first portion to a second side of the first portion and a light source housed within the main body comprising a first set of lights and a second set of lights, wherein each of the first and second set of lights are operatively connected to the frame and configured to emit light into the first portion and through the set of passageways defined by the first portion.

In some embodiments, the first set of lights comprises at least one LED light.

In some embodiments, the LED light is configured to emit red light and/or blue light.

In some embodiments, the LED light is configured to emit one or more of yellow, purple, cyan, orange, and/or green light.

In some embodiments, the first portion is transparent.

In some embodiments, the second sight of lights comprises at least one NIR-emitting light.

In some embodiments, the at least one NIR-emitting light is disposed adjacent the first end of the device.

In some embodiments, each passageway of the first set of passageways is substantially frustum-shaped.

In some embodiments, each passageway of the first set of passageways has a first opening and a second opening; and wherein each of the first opening and the second opening is substantially circular in shape.

In some embodiments, each passageway of the first set of passageways defines a height; and wherein the height is between about 1mm and about 5 mm.

In some embodiments, each passageway of the first set of passageways defines a width; and wherein the width is between about 0.5 mm and about 4 mm.

In some embodiments, the treatment device is a treatment mask configured to be used adjacent a head of a user, and further comprising a strap attached to the main body, the strap being configured to secure said treatment mask to said head of said user.

In some embodiments, the first set of lights comprises at least twenty LED lights.

In some embodiments, each of the LED lights is configured to emit red and/or blue light.

In some embodiments, the second set of lights comprises at least four NIR-emitting lights.

In some embodiments, the at least one NIR-emitting light is disposed adjacent the first end of the device.

In some embodiments, the frame is a plastic frame.

In some embodiments, the first portion is a silicon portion.

In some embodiments, the set of passageways is a set of conical passageways defined by the silicone portion.

In some embodiments, the treatment device further comprises a silicone strap attached to the main body, the strap being configured to secure said treatment mask to said head of said user.

In some embodiments, the first set of lights comprises at least forty LED lights.

In some embodiments, each of the LED lights is configured to emit red and/or blue light.

In some embodiments, the frame is coated in a metal.

Another example treatment mask configured to be used adjacent a head of a user, comprises a main body having a first end, a second end substantially opposite the first end, an intermediate portion extending from the first end to the second end, a frame, and a first portion disposed secured to the frame, the first portion defining a set of passageways extending from a first side of the first portion to a second side of the first portion, a light source housed within the main body comprising a first set of lights and a second set of lights, wherein each of the first and second set of lights are operatively connected to the frame and configured to emit light into the first portion and through the set of passageways defined by the first portion, and a strap attached to the main body, the strap being configured to secure said treatment mask to said head of said user.

In some embodiments, the first set of lights comprises at least twenty LED lights.

In some embodiments, each of the LED lights is configured to emit red and/or blue light.

In some embodiments, the second sight of lights comprises at least four NIR-emitting lights.

In some embodiments, the at least one NIR-emitting light is disposed adjacent the first end of the device.

Another example treatment device comprises a main body having a first end, a second end substantially opposite the first end, an intermediate portion extending from the first end to the second end, a plastic frame, and a silicone portion disposed secured to the frame, the silicone portion defining a set of passageways extending from a first side of the silicone portion to a second side of the silicone portion, a light source housed within the main body comprising a first set of lights and a second set of lights, wherein each of the first and second set of lights are operatively connected to the frame and configured to emit light into the silicone portion and through the set of conical passageways defined by the silicone portion, and a silicone strap attached to the main body, the strap being configured to secure said treatment mask to said head of said user.

In some embodiments, the first set of lights comprises at least forty LED lights.

In some embodiments, each of the LED lights is configured to emit red and/or blue light.

In some embodiments, the frame is coated in a metal.

Additional understanding of claimed devices and methods may be obtained by reviewing the detailed description of selected examples, below, with reference to the appended drawings.

### DESCRIPTION OF FIGURES

FIG. 1 is a perspective view of a first example light treatment device.
FIG. 2 is an end view of the light treatment device illustrated in FIG. 1.
FIG. 3 is another end view of the light treatment device illustrated in FIG. 1, in which various portions of the device are magnified.
FIG. 4 is another end view of the light treatment device illustrated in FIG. 1, with a portion of the device having been removed.
FIG. 5 is another side view of the light treatment device illustrated in FIG. 1.
FIG. 6 is another side view of the light treatment device illustrated in FIG. 1.
FIG. 7 is a top view of the light treatment device illustrated in FIG. 1.
FIG. 7A is a top view of the light treatment device illustrated in FIG. 7, with a portion of the silicone of the device removed in order to clearly illustrate various LED lights.
FIG. 8 is a bottom view of the light treatment device illustrated in FIG. 1.
FIG. 8A is a top view of the light treatment device illustrated in FIG. 8, with a portion of the silicone of the device removed in order to clearly illustrate various LED lights.
FIG. 9 is a diagram illustrating components of a networked light treatment device in an example embodiment.
FIG. 10 is a flowchart representation of an example method of using a light treatment device.

The figures depict various embodiments of the present invention for purposes of illustration only. One skilled in the art will readily recognize from the following discussion that alternative embodiments of the structures and methods illustrated herein may be employed without departing from the principles of the invention described herein.

### DETAILED DESCRIPTION OF SELECTED EXAMPLES

The following detailed description and the appended drawings describe and illustrate various light treatment devices and methods of use. The description and drawings are provided to enable one skilled in the art to make and use one or more example light treatment devices. They are not intended to limit the scope of the claims in any manner.

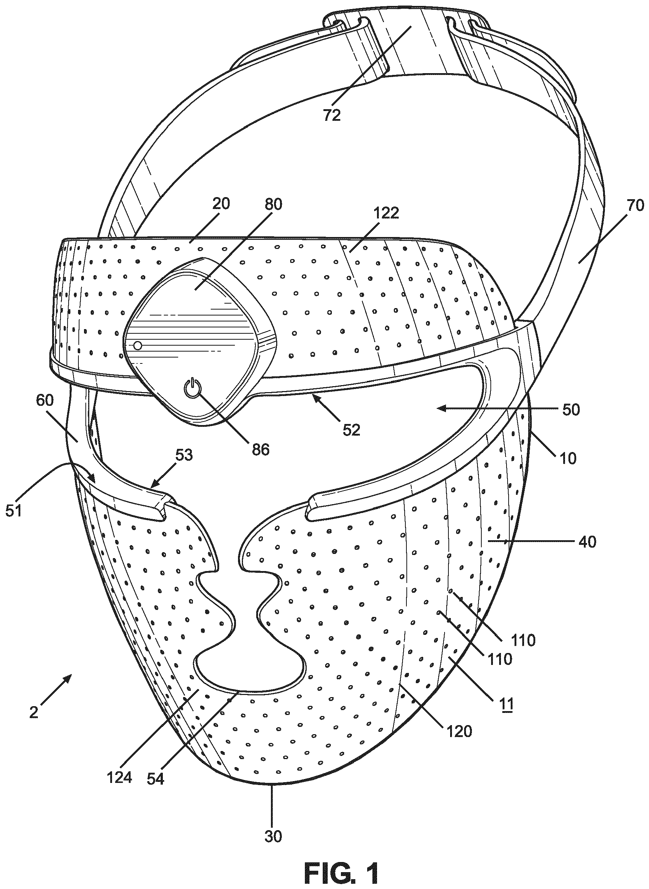

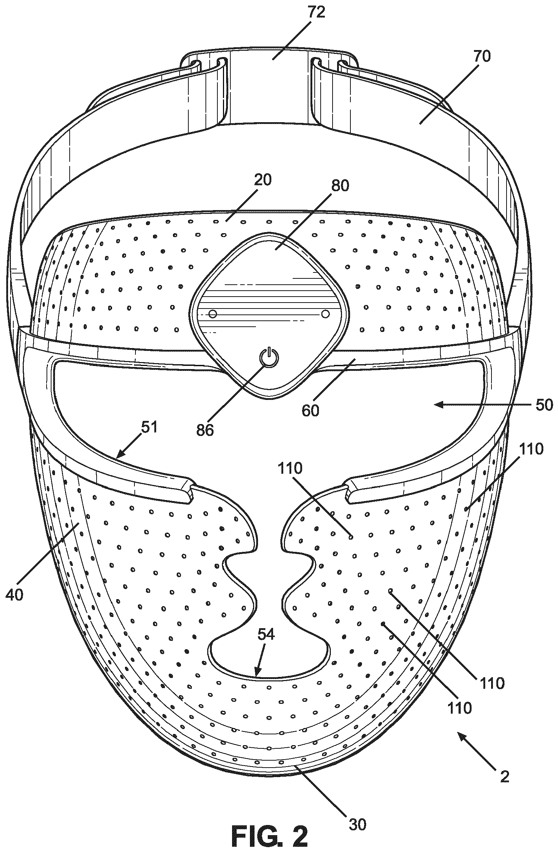

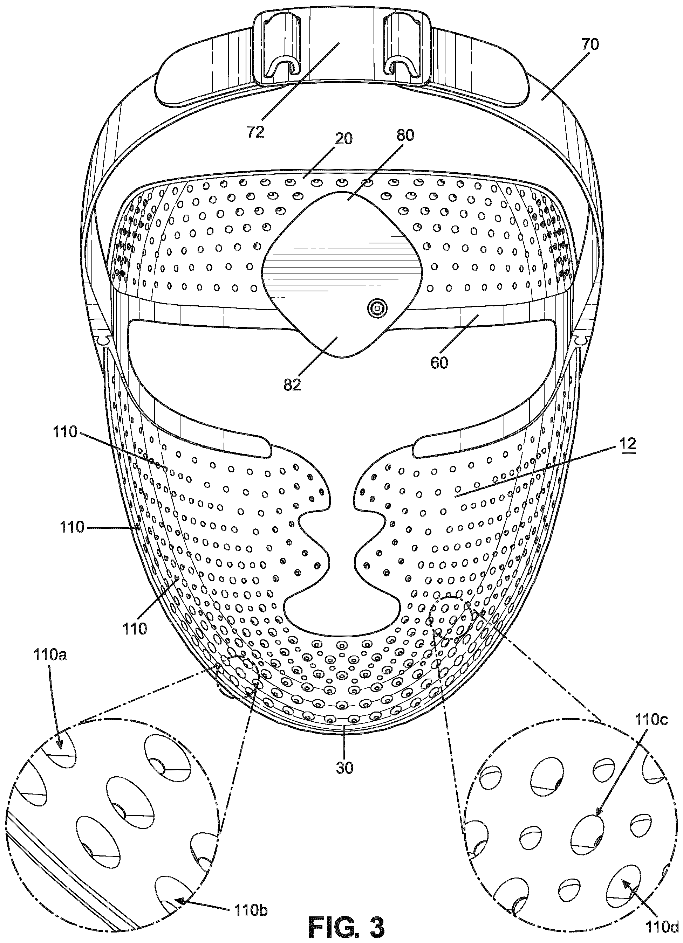

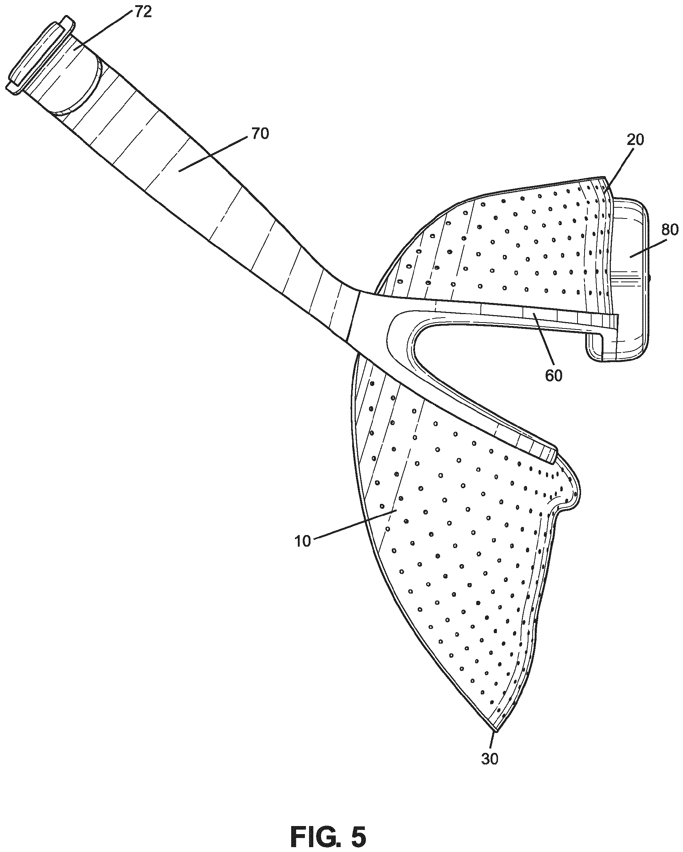

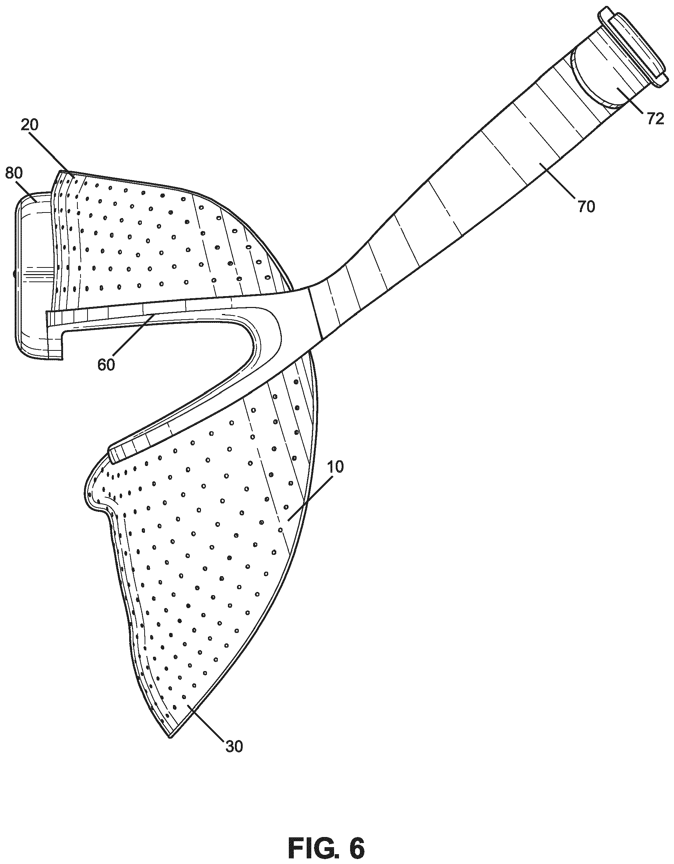

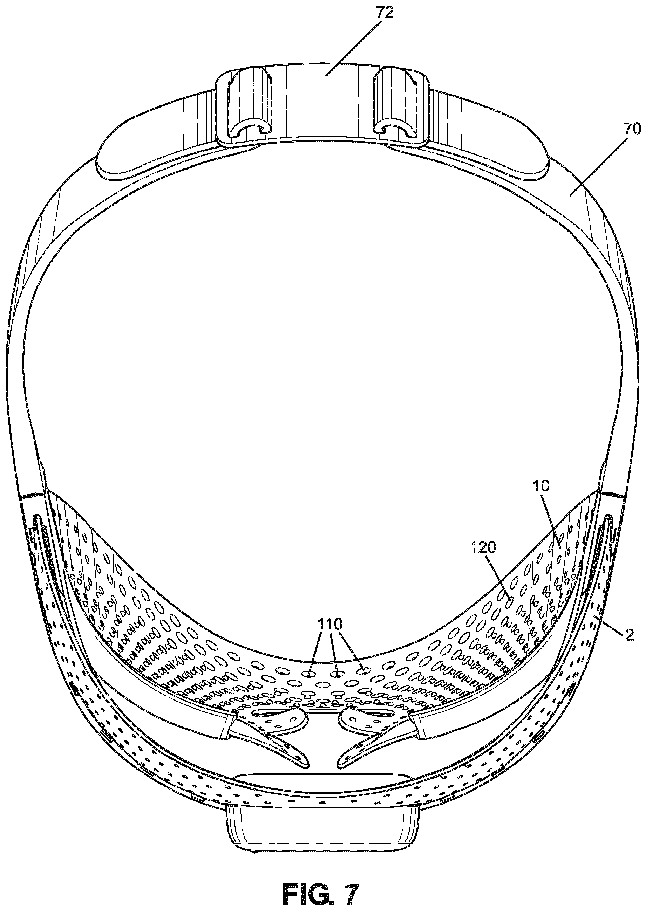

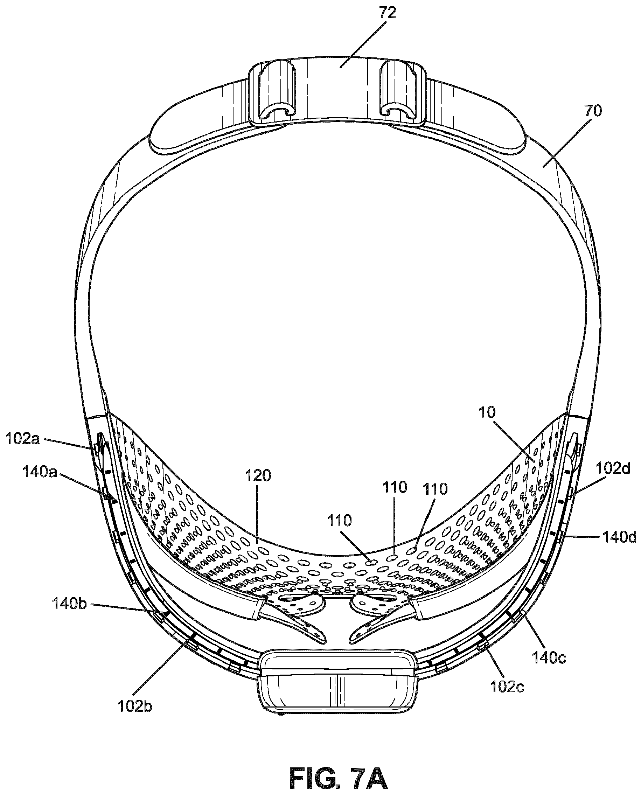

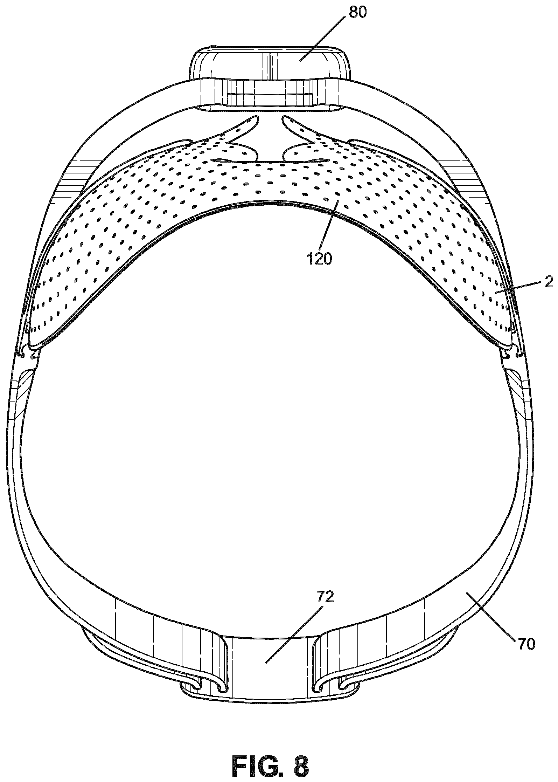

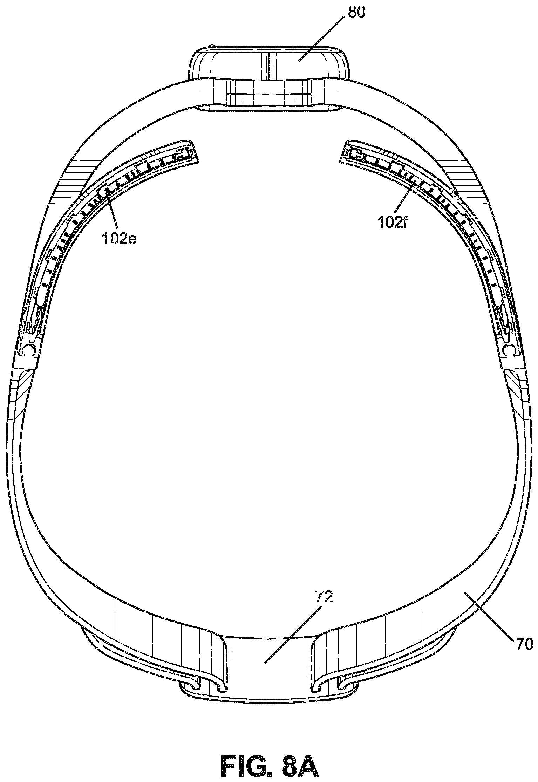

FIGS. 1, 2, 3, 4, 5, 6, 7, 8, and 9 illustrate an example light treatment device 2 (hereinafter, also referred to as the "device"). The device 2 comprises a main body 10 that includes and/or houses several components (described in greater detail below).

The main body 10 includes at least a first end 20, a second end 30 substantially opposite the first end 20, and an intermediate portion 40 extending from the first end 20 to the second end 30. More specifically, the main body 10 is configured such that a user can wear the device 2 in order to provide light therapy treatment to the areas of his or her body that are in contact with or adjacent to the device 2.

The main body 10 is shaped such that a user of the device will wear it as a mask that covers most of the face of the user. The main body 10 will cover, generally, the forehead, cheeks, jawline, chin, brows, and various other portions of the face when it is worn. The main body 10, however, defines a passageway 50 extending from the first surface 11 of the main body 10 to the second surface 12 of the main body 10. The passageway 50 is shaped such that the various portions of the face are not covered by the main body 10. The upper portion 52 of the passageway 50 is the widest portion of the passageway 50. It generally is positioned such that the eyes of the user align with the passageway 50 and, hence, are not directly contacted by the light emitted by the light source 100. The passageway 50 narrows as it approaches the lower portion 54 of the passageway 50, though not in a uniform manner. Specifically, the passageway 50 includes a portion that narrows prior to reaching the portion of the passageway 50 through which a user's nose will extend, then expands, and then narrows again beneath the portion of the passageway 50 through which a user's nose will extend. Additionally, the passageway 50 then expands again in width as it approaches the lower portion 54, which is configured to create an opening that will surround a user's lips. A skilled artisan will be able to determine how best to form the main body and passageway according to a particular example based on various considerations, including the desired flexibility of the device, the size and shape of the head of a potential user, and the functionality of the device. In other embodiments, the main body may not have a passageway. In another embodiment, zero, two, three, or four openings may be formed by the main body which may correspond to one or more of the lips, nose, mouth and eyes, and may be adjacent, integrally formed with, connected to, or totally separate from any other opening. A skilled artisan will be able to determine how best to size and shape the device by determining optimal sizes and shapes for the portion of the passageway that is configured to be disposed near or about a user's eyes and/or his or her nose and/or his or her mouth. The device should be sized and shaped such that it is able to be worn by a variety of users having different sizes and proportions.

The main body 10 includes a frame and a portion of the device through which light is emitted to treat the skin. The frame is described in greater detail, below. The portion of the device through which light is emitted to treat the skin is at least partially comprised of silicone in the illustrated embodiment. This portion, referenced in the following paragraphs as a silicone portion 120 when referring to the illustrated embodiment is medical grade and is safe for a user to touch and/or wear, and can be heated as needed. The silicone portion 120 is configured to roughly cover the face of most users (though, of course, face and head sizes and shapes can differ greatly by person). The silicone portion 120 has a first end 122, a second end 124, and comprises first 11 and second 12 surfaces.

The light source 100 (discussed in greater detail, below) is disposed adjacent to and operatively connected to a frame 60 (which is adjacent the passageway 50) and between the first 11 and second 12 surfaces of the silicone portion 120. Specifically, the frame 60 is comprised of plastic and is configured to extend along the exterior opening 51 and interior opening 53 of the passageway 50. The frame 60 is configured to touch the skin of a user when the device 2 is in use. A skilled artisan will be able to best select materials for the main body and frame according to a particular example based on various considerations, including the size and shape of the main body, the desired functionality of the main body, and the desired passageway size and shape. In another embodiment, the devices may not have a frame; it also may have more than one frame, and the frame may be disposed upon any portion of the main body. Additionally, the frame and/or main body may be comprised of silicone, plastic, metal, and/or any other suitable material in different embodiments. The frame may also be coated with gold, gold imitation, or similar plating. The portion through which light is emitted to treat the skin may also have any size and shape, and in other embodiments may be comprised of material other than silicone. Other such embodiments may include portions through which light is emitted to treat the skin that are comprised of plastic-Other such embodiments may include portions through which light is emitted to treat the skin comprising transparent, partially transparent, or semitransparent material that is conducive to transmitting light to a user's skin. Additionally, the silicone portion and frame may be connected to one another by one or more adhesives, one or more physical attachment mechanism, being integrally formed, and/or via any other means.

The frame 60, and thus main body 10, are also attached to a strap 70. The strap 70 is operatively connected to the frame 60 and main body 10 via a mechanical attachment. The strap 70 is configured to be wrapped around and secured to a user's head, after the device 2 is placed on the user, and can be tightened or loosened, depending on the size and shape of the user's head. The strap 70 includes a clasp 72 that allows for the clasp 72 to be tightened and loosened by inserting more or less of the strap 70 into the clasp 72. The strap 70 is comprised of silicone in the illustrated embodiment. A skilled artisan will be able to select a suitable strap and clasp according to a particular example based on various considerations, including the size and shape of the main body, whether the strap is flexible (i.e., can be tightened and loosened), and/or the desired output of the device. In other embodiments, zero, two, three, or more than three straps and/or clasps may be used. In different embodiments, the strap is integrally with the main body; it may also be adhesively attached to or molded to the main body. The strap may also be made of any suitable material in alternative embodiments, including leather, metal, or any other suitable material.

The main body 10, as noted above, also includes a light source 100 disposed within the main body 10, between its first 11 and second 12 surfaces. More specifically, the light source 100 includes a series of light-emitting diodes ("LED lights") housed within the main body 10 and operatively connected to the frame 60 and control panel (described below). The LED lights 102a, 102b, 102c, 102d, 102e, 102f and the like (only some of which include reference numbers; others are visibly illustrated and similar in size, shape, and functionality of those which are numbered) are configured to emit light in order to treat a user's face. Specifically, the LED lights are configured to emit various types of light, depending on a user's preference. In the illustrated embodiment, the LED lights 102a, 102b, 102c, 102d, 102e, 102f are configured to emit blue, red, yellow, orange, purple, green and/or cyan colored light. A user can toggle through controls on the device 2 to determine which type(s) of light he or she would like the device 2 to emit at a particular time. In other embodiments, the device 2 can emit one, two, three, or more than three of of aforesaid colored LED light; it may also light in colors not described in this application. The light source 100 may include any satisfactory number of light sources; it may also include various non-LED light sources, such as near infrared light sources. In various embodiments, the device may emit light, via LED, NIR, or any other type of light, having wavelengths between about 100 nanometers ("nm") and about 900 nm, between about 200 nm and about 800 nm, between about 300 nm and about 700 nm, or any other suitable wavelength.

The light source 100 includes LED lights 102a, 102b, 102c, 102d, 102e, 102f which are adjacent dozens of passageways 110 extending from the first surface 11 to the second surface 12 of the device 2. A number of said passageways 110a, 110b, 110c, 110d are described by reference number in this embodiment, though other such passageways 110 are clearly visible in the drawings. When in use and the device 2 is turned on, light is able to be emitted via the main body 10 to the user because the light travels through and adjacent the silicone portion of the device 2 and is emitted through the passageways 110 and onto a user's skin.

Additionally, four NIR lights 140 are also operatively connected and adjacent to the frame 60. The first NIR light 140a and second NIR light 140b are disposed adjacent and in contact with the frame 60 such that they will emit light to treat the right portion of a user's face. The third NIR light 140c and fourth NIR light 140d are disposed adjacent and in contact with the frame 60 such that they will emit light to treat the left portion of a user's face. The NIR lights 140 are disposed along the top portion 62 of the frame 60 and are not directly adjacent one another; rather, various other lights are placed between any two of the NIR lights. They are configured to treat the forehead and area's around a user's eyes when in use. Like the light source 102 described above, they are attached to the frame 60 and emit light via the passageways 110 surrounding the NIR lights 140 and throughout the device 2.

The passageways 110 allow for the light emitted by the light source 100 to safely contact a user's skin in order to treat the user. The passageways 110 have various diameters; those which are closer to an LED light tend to have smaller diameters than those which are further from an LED light, though this is a general description and not necessary in each alternative embodiment. Some passageways 110 will have the same diameter as other passageways 110, of course. The diameter of each passageway 110 is between .1 millimeters and 1 centimeter. Each passageway is substantially frustum-shaped, with circular openings at each end of each of the passageways.

A skilled artisan will be able to select a suitable frame, set of LED and/or NIR lights, light source, strap, and clasp according to a particular example based on various considerations, including the size and shape of the main body, the number of light sources used, and the number of passageways used. In one embodiment, fifty LED lights may be used. In others, fewer than 10, 10 to 20, 20 to 50, or more than 50 LED lights may be used. Additionally, in different embodiments, zero, one, two, three, four, five, or more than five NIR light sources may be used. The passageways, in various embodiments, may have any size, shape, number, and configuration. They may be circular, triangular, conical, cylindrical, oval, elliptical, square, rectangular, or have any other shape. Additionally, the light source and LED lights may be placed on any portion of the main body in other configurations. Any individual light may emit one or more than one such type of light or an individual light color or type, in various embodiments. Moreover, each of the passageways may have openings on the first surface of the silicone portion that have widths between about 0.8 millimeters ("mm") and about 4 mm, between about 1 mm and about 3.4 mm, and/or between about 1.3 mm and about 2.8 mm. Additionally, each of the passageways may have heights between about 1.2 millimeters ("mm") and about 5 mm, between about 1 mm and about 4.8 mm, and/or between about 1.5 mm and about 3.5 mm. The passageways, of course, may have different heights and opening widths or equal heights and opening widths.

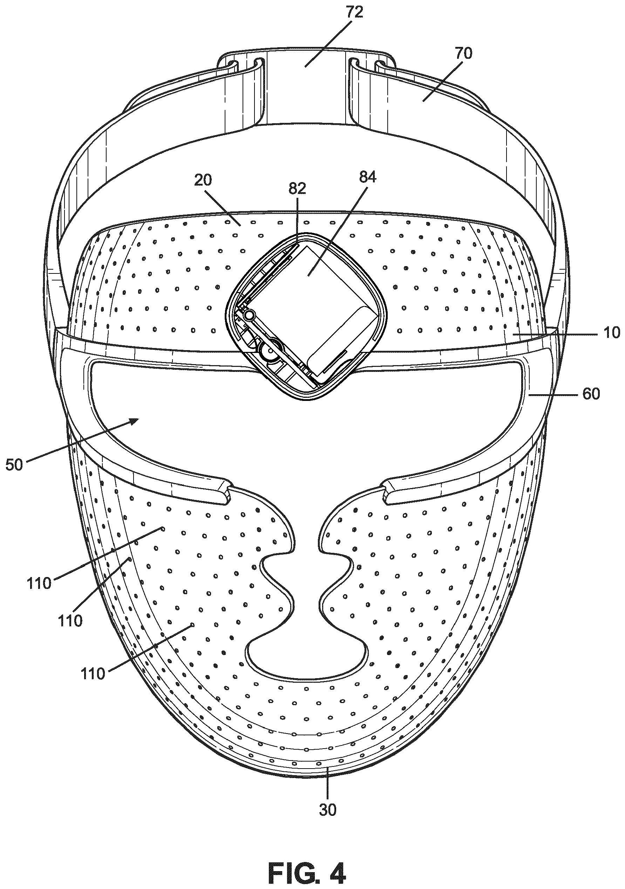

The main body 10 also includes a control panel 80 disposed on the front of the device 2. The control panel 80 is substantially diamond shaped and includes a controller 82 and a power source 84 housed within the control panel 80. FIG. 4 illustrates the control panel 80 with its front portion removed, such that the interior of the control panel 80 is visible.

The power source 84 comprises a rechargeable battery that provides power to the controller 82. The controller 82 is operatively connected to each of the light source 100 and power source 84 and provides instructions to and controls each of these components. The controller 82 is activated by a user control 86, visible on the exterior of the control panel 80. The controller 82 in the illustrated embodiment includes a printed circuit board assembly ("PCBA") and related circuitry; however, in other embodiments, the controller may comprise any device suitable to control the skincare device's components, such as a printed circuit board ("PCB") or any other suitable computer or mechanism for communicating information. Additionally, the controller 82 may be operatively connected to an interface, allowing it to communicate with a second device (described in greater detail, below).

The user control 86 comprises a button which, when pressed, activates the device 2. Repeated and/or a specific type of use of the user control 86 allows for a user to cycle through various modes of operation of the device 2 (described in greater detail, below). Various types and patterns of light emissions may be selected by the user via the user control 80. A skilled artisan will be able to suitably place the user control on the device and configure the same according to a particular example based on various considerations, including the number of desired modes and the materials comprising the main body. In other embodiments, the user control may be disposed on any side of the main body. In a different embodiment, the device may comprise zero, two, three, or more than three user controls. In additional embodiments, the user control may include separate user controls configured to control individual components and/or actions.

The controller 82, optionally, also controls an interface 90 that is a component of the controller 82. The interface 90 allows the device 2 to communicate with a second device, such as a personal computer, tablet, mobile telephone, or other electronic device (not illustrated in the Figures). Using the interface 90, the device 2 can send information to other devices so that the other device(s) may collect data pertaining to the use of the device 2. Additionally, the device 2 may receive control signals from another device that can indicate that the device 2 should turn on or off, increase or decrease light emissions, and/or switch to a pre-set pattern desired by the user or recommended by the other device, among other instructions. The interface 90 can be a wired or a wireless interface, such as a wireless transceiver that transmits control signals between the device 2 and the second device. A skilled artisan will be able to select a suitable interface based on various considerations, including the device with which the skincare device will communicate and the size and shape of the main body. In some embodiments, the interface is a radiofrequency ("RF") transceiver used to transmit and receive RF signals between the skincare device and other devices. One example of an RF transceiver that could be used is a low power 2.4 GHz RF transceiver. In various embodiments, the device may also include antennas for transmitting and receiving signals between the device and other devices. In such examples, the interface can use BLUETOOTH^{®}, Wi-Fi, infrared, laser light, visible light, acoustic energy, or one of a number of other methods to transmit information wirelessly between the skincare device and another device.

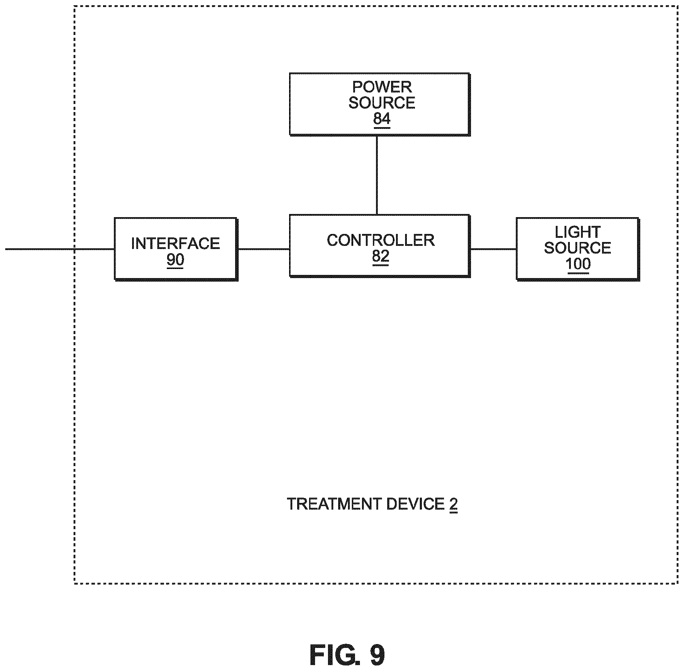

In some embodiments, the device is connected to a network via the second device. In other embodiments, the skincare device is directly connected to a wireless router or cellular phone network and may connect with the second device in any of said manners. Accordingly, the skincare device can be controlled via personal computer, tablet, mobile phone, or other suitable electronic devices a user using the personal computer, tablet, phone, or other device. FIG. 9 illustrates one example of such a design.

FIG. 9 is a diagram illustrating components of a networked skincare device, such as device 2, in accordance with an example embodiment. In this embodiment, the device includes a controller, such as controller 82, a light source, such as light source 100, and an interface, such as interface 90. As explained above, the device can be connected to a network via a personal computer, tablet, mobile telephone, or other electronic device or can be directly connected to a wireless router or cellular phone network. Thus, the device can be controlled by, transmit data to, and/or receive data from the personal computer, tablet, mobile telephone, or other electronic device via the aforementioned mechanisms. The interface may be wired or wireless and may include any of those described above. A skilled artisan will be able to determine how to suitably connect the device with other devices based on various considerations, including the desirability of doing so and the devices to which connection would be beneficial. In some embodiments, the skincare device may not include an interface and, thus, may not communicate with other devices. In different embodiments, the skincare device may only transmit data to other devices; it may not receive any data and cannot be controlled via said other devices in this embodiment. In a different embodiment, the controller does not communicate with external apparatuses outside of the device.

Example data that the device may communicate to one or more of a personal computer, tablet, mobile telephone, or other electronic device may include the number of uses of the device, the durations of the various uses of the skincare device, the user's preferred device settings, and various other types of information related to the use of the device.

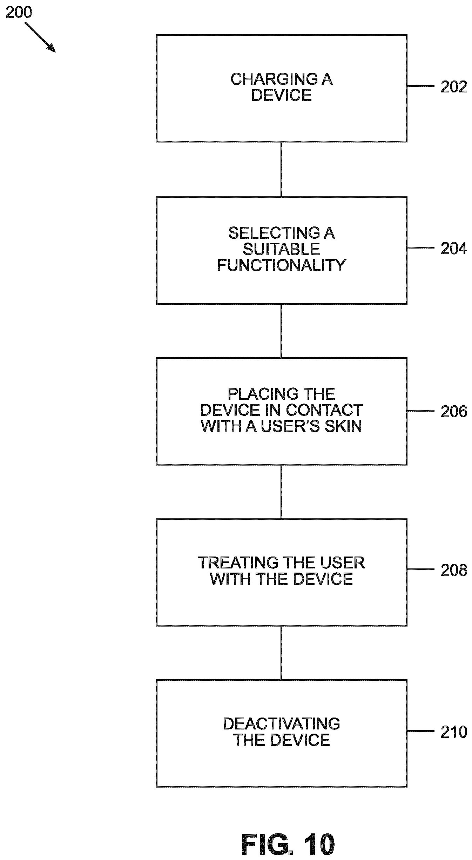

FIG. 10 is a flowchart representation of an example method 200 of using a light treatment device. Performance of this method results in the treatment of the user's body, such as the face and/or neck, by a treatment device.

An initial step 202 comprises charging a device, such as device 2 by connecting the device to a power source via a DC jack.

Another step 204 comprises selecting a suitable light treatment functionality. A treatment may be selected via the user control. Selection of a particular treatment pattern may be communicated to a user via informational lights, which may emit different patterns, colors, or flashes of light to indicate the various treatment patterns.

Another step 206 comprises placing the treatment device in contact with or adjacent the user. Doing so allows the treatment device to treat the skin via the selected treatment functionality.

Another step 208 comprises treating the user with the device.

Optionally, another step 210 comprises deactivating the device. Deactivation of the device may occur automatically at the end of a pre-set treatment or via manipulation of the user control.

It is noted that it is advantageous to complete this method 200 in the order illustrated and described. However, any order is considered suitable.

In all examples, a device and its various components may be formed of any suitable material, including presently known and later-developed materials. A skilled artisan will be able to select appropriate materials for an example treatment device based on various considerations, including the size and shape of the treatment device, the motor housed within the treatment device, the light source housed within the treatment device, and the particular treatments desired.

Those with ordinary skill in the art will appreciate that various modifications and alternatives for the described and illustrated embodiments can be developed in light of the overall teachings of the disclosure. Accordingly, the particular arrangements disclosed are intended to be illustrative only and not limiting as to the scope of the invention, which is to be given the full breadth of the appended claims and any and all equivalents thereof.

## Claims

1. A treatment device configured to be worn by a user, comprising:
a main body having a first end, a second end substantially opposite the first end, an intermediate portion extending from the first end to the second end, a frame, and a first portion secured to the frame, the first portion defining a set of passageways extending from a first side of the first portion to a second side of the first portion; and
a light source housed within the main body comprising a first set of lights and a second set of lights, wherein each of the first and second set of lights are operatively connected to the frame and configured to emit light into the first portion and through the set of passageways defined by the first portion.

2. The treatment device of claim 1, wherein the first portion is transparent.

3. The treatment device of any one of the preceding claims, wherein each passageway of the first set of passageways is substantially frustum-shaped.

4. The treatment device of any one of the preceding claims, wherein each passageway of the first set of passageways has a first opening and a second opening; and
wherein each of the first opening and the second opening is substantially circular in shape.

5. The treatment device of any one of the preceding claims, wherein each passageway of the first set of passageways defines a height; and
wherein the height is between about 1mm and about 5 mm.

6. The treatment device of any one of the preceding claims, wherein each passageway of the first set of passageways defines a width; and
wherein the width is between about 0.5 mm and about 4 mm.

7. The treatment device of any one of the preceding claims, the treatment device being a treatment mask configured to be used adjacent a head of a user, and further comprising:
a strap attached to the main body, the strap being configured to secure said treatment mask to said head of said user.

8. The treatment device of any one of the preceding claims, configured to be used adjacent a head of a user, wherein the frame is a plastic frame, the first portion is a silicon portion and the set of passageways is a set of conical passageways defined by the silicone portion; and the treatment device further comprises:
a silicone strap attached to the main body, the strap being configured to secure said treatment mask to said head of said user.

9. The treatment device of any one of the preceding claims, wherein the first set of lights comprises at least one LED light.

10. The treatment device of any one of the preceding claims, wherein the first set of lights comprises at least twenty or optionally at least forty LED lights.

11. The treatment device of claim 9 or claim 10, wherein each of the LED lights is configured to emit red and/or blue light.

12. The treatment device of claim 9, claim 10 or claim 11, wherein the or each LED light is configured to emit one or more of yellow, purple, cyan, orange, and/or green light.

13. The treatment device of any one of the preceding claims, wherein the second set of lights comprises at least one NIR emitting light, and optionally at least four NIR-emitting lights.

14. The treatment device of claim 13, wherein the at least one NIR-emitting light is disposed adjacent the first end of the device.

15. The treatment device of any one of the preceding claims, wherein the frame is coated in a metal.
